# EUROPEAN PATENT SPECIFICATION

(11) **EP 0 689 456 B1**
(45) Date of publication and mention of the grant of the patent: **07.11.2001**
(21) Application number: 94907392.8
(22) Date of filing: 01.02.1994
(51) Int. Cl.: A61K 47/00, A61K 7/00, A61K 7/48

(54) **COSMETIC COMPOSITIONS**
KOSMETISCHE MITTEL
COMPOSITIONS COSMETIQUES

(30) Priority: 09.02.1993 GB 9302492
(43) Date of publication of application: 03.01.1996
(73) Proprietor: THE PROCTER & GAMBLE COMPANY, Cincinnati, Ohio 45202 (US)
(72) Inventor: DATE, Robert Francis, Surrey GU21 1QB (GB); COURPRON, Elise, F-75116 Paris (FR); NAWAZ, Zahid, High Wycombe Buckinghamsire HP13 5SJ (GB); ROLLS, Richard George A., Middlesex TW14 9HL (GB)
(74) Representative: Brooks, Maxim Courtney
(86) International application number: US9401135
(87) International publication number: WO9417830

(56) References cited:
- EP-A- 0 458 600
- DATABASE WPI Week 7833 Derwent Publications Ltd., London, GB; AN 78-59474a XP002011710 "Hand cream based on edible components - which are sucrose fatty acid ester, edible animal or vegetable oil and fat, water and emulsifier" & JP-A-53 079 043 (DAIICHI KOGYO) , 13 July 1978
- CHEMICAL ABSTRACTS, Volume 112, No. 22, issued 1989, KITAHARA et al., "Oil-in-Water and Oil-in-Polyhydric Alcohol Emulsions Containing Sucrose Fatty Acid Esters and Polyhydric Alcohols", Abstract No. 204452q; & JP,A,01 176 444.
- CHEMICAL ABSTRACTS, Volume 95, No. 14, issued 1981, Nihon Surfactants Industry Co., Ltd., "Water-in-Oil Emulsifiers for Cosmetic Creams", Abstract No. 120983f; & JP,A,56 024 034.
- CHEMICAL ABSTRACTS, Volume 102, No. 24, issued 1985, ONDRACEK et al., "Comparison among Five Methods for Testing the Physical Stability of O/W Emulsions", Abstract No. 209299y, Acta Pharm. Technol., 31(1), 42-8.
- CHEMICAL ABSTRACTS, Volume 117, No. 4, issued 1991, TSUDA et al., "Transparent or Semitransparent Hair Preparations Containing Surfactants, Oils, and/or Fats, and Water-Soluble Alcohols", Abstract No. 33422b; & JP,A,03 223 208.

## Description

### Technical Field

The present invention relates to cosmetic compositions. In particular it relates to cosmetic compositions in the form of emulsions or lotions which provide improved moisturization, skin feel and skin care benefits and reduced greasiness, together with excellent rub-in and absorption characteristics. The compositions also display excellent stability characteristics at normal and elevated temperatures.

### Background of the Invention

Skin is made up of several layers of cells which coat and protect the keratin and collagen fibrous proteins that form the skeleton of its structure. The outermost of these layers, referred to as the stratum corneum, is known to be composed of 25nm protein bundles surrounded by 8nm thick layers. Anionic surfactants and organic solvents typically penetrate the stratum corneum membrane and, by delipidization (i.e. removal of the lipids from the stratum corneum), destroy its integrity. This destruction of the skin surface topography leads to a rough feel and may eventually permit the surfactant or solvent to interact with the keratin, creating irritation.

It is now recognised that maintaining the proper water gradient across the stratum corneum is important to its functionality. Most of this water, which is sometimes considered to be the stratum corneum's plasticizer, comes from inside the body. If the humidity is too low, such as in a cold climate, insufficient water remains in the outer layers of the stratum corneum to properly plasticize the tissue, and the skin begins to scale and becomes itchy. Skin permeability is also decreased somewhat when there is inadequate water across the stratum corneum. On the other hand, too much water on the outside of the skin causes the stratum corneum to ultimately sorb three to five times its own weight of bound water. This swells and puckers the skin and results in approximately a two to three fold increase in the permeability of the skin to water and other polar molecules.

Thus, a need exists for compositions which will assist the stratum corneum in maintaining its barrier and water-retention functions at optimum performance in spite of deleterious interactions which the skin may encounter in washing, work, and recreation.

Conventional cosmetic cream and lotion compositions as described, for example, in Sagarin, Cosmetics Science and Technology, 2nd Edition, Vol.l, Wiley Interscience (1972) and Encyclopaedia of Chemical Technology, Third Edition, Volume 7 are known to provide varying degrees of emolliency, barrier and water-retention (moisturizing) benefits. However, they can also suffer serious negatives in terms of skin feel (i.e. they often feel very greasy on the skin) as well as having poor rub-in, absorption and residue characteristics.

Abstract no. 78-59474a (Derwent Publications Ltd., London, GB) & JP-A-53 079 043, 1978 refer to a hand cream, comprising an emulsifier selected from i.a. sorbitan fatty acid ester and sucrose fatty acid ester.

EP-A-0 458 600 discloses topical cosmetic emulsions consisting i.a. of special esters, resulting in a water vapour retarding layer to reduce moisture loss from the skin.

The present invention provides skin-care cosmetic compositions which provide improvements in moisturization, absorption, skin feel and skin care characteristics and which in particular provide improved short and longer term moisturizing effectiveness, while at the same time reducing stickiness and avoiding a greasy feel on the skin. The compositions also display excellent stability characteristics at both normal and elevated temperatures.

### Summary of the Invention

Accordingly, in one aspect of the present invention, there is provided a skin care composition in the form of an oil-in-water dispersion comprising one or more distinct oil phases, wherein the primary oil phase is present in a level of from 4% to 16% by weight and wherein the composition incorporates from 2% to 10% of an emulsifier capable of forming liquid crystals in water, the emulsifier being a fatty acid ester blend based on a mixture of sorbitan fatty acid ester and sucrose fatty acid ester, and wherein the ratio of primary oil phase to fatty acid ester emulsifier is in the range from 6:1 to 1:1.

The compositions of this aspect of the present invention take the form of an oil-in-water emulsion or dispersion containing one or more distinct emulsified or dispersed oil phases and an essential emulsifier component as well as various optional ingredients as indicated below. All levels and ratios are by weight of total composition, unless otherwise indicated. Chain length and degrees of ethoxylation are also specified on a weight average basis.

According to the first aspect of the invention, a first essential component of the composition herein is an oil phase, referred to herein as a primary oil phase, which can comprise a single oily component or a mixture of oily components in miscible or homogeneous form. This is preferably present in an amount of from 4% to 16%, more preferably from 5% to 11% by weight of composition. The level of primary oil phase component is found to be important herein for achieving optimum moisturization and greasiness characteristics. The primary oil phase generally comprises a natural or synthetic oil selected from mineral, vegetable, and animal oils, fats and waxes, fatty acid esters, fatty alcohols, fatty acids and mixtures thereof having emollient cosmetic properties. The primary oil phase component is preferably essentially silicone-free, ie contains no more than 10%, preferably no more than 5% by weight of silicon-based materials. It will be understood that the oil phase may contain small levels (eg. up to 25%, preferably 10%) of oil phase soluble emulsifier ingredients. Such ingredients are not to be considered as oil phase components from the viewpoint of determining the oil phase level and required HLB. In preferred embodiments, the overall required HLB of the oil phase is from 8 to 12, especially from 9 to 11, required HLB being determined by summing the individual required HLB values for each component of the oil phase multiplied by its W/W percentage in the oil phase (see ICI Literature on HLB system).

Suitable primary oil phase components for use herein include, for example, optionally hydroxy-substituted C₈-C₅₀ unsaturated fatty acids and esters thereof, C₁-C₂₄ esters of C₈-C₃₀ saturated fatty acids such as isopropyl myristate, cetyl palmitate and octyldodecylmyristate (Wickenol 142), beeswax, saturated and unsaturated fatty alcohols such as behenyl alcohol and cetyl alcohol, hydrocarbons such as mineral oils, petrolatum and squalane, fatty sorbitan esters (see US-A-3988255, Seiden, issued October 26 1976), lanolin and lanolin derivatives, animal and vegetable triglycerides such as almond oil, peanut oil, wheat germ oil, linseed oil, jojoba oil, oil of apricot pits, walnuts, palm nuts, pistachio nuts, sesame seeds, rapeseed, cade oil, corn oil, peach pit oil, poppyseed oil, pine oil, castor oil, soybean oil, avocado oil, safflower oil, coconut oil, hazelnut oil, olive oil, grapeseed oil, and sunflower seed oil and C₁₋C₂₄ esters of dimer and trimer acids such as diisopropyl dimerate, diisostearylmalate, diisostearyldimerate and triisostearyltrimerate. Of the above, highly preferred are the mineral oils, petrolatums, unsaturated fatty acids and esters thereof and mixtures thereof.

Compositions herein preferably also comprise a secondary oil phase which in preferred embodiments is present in a level of from about 0.1% to about 20%, especially from about 1% to about 10% by weight of composition. Moreover, the primary oil phase is preferably present in weight excess of the secondary oil phase. The secondary oil phase component is preferably silicone-based. Suitable silicone components herein include water-insoluble silicones inclusive of non-volatile polyalkyl and polyaryl siloxane gums and fluids, volatile cyclic and linear polyalkylsiloxanes, polyalkoxylated silicones, amino and quaternary ammonium modified silicones, rigid cross-linked and reinforced silicones and mixtures thereof. In preferred embodiments the silicone component is a silicone gum having a molecular weight of from about 200,000 to about 4,000,000 or a mixture of silicones including the silicone gum. In mixtures, the silicone gum preferably constitutes from about 5% to about 40%, especially from about 10% to 20% by weight of the silicone mixture. The silicone or silicone mixture preferably constitutes from about 0.1% to about 20%, more preferably from about 0.5% to about 15%, and especially from about 1% to about 10% by weight of composition.

A preferred silicone component for use herein consists essentially of:
(i) a silicone having a molecular weight of from 200,000 to 4,000,000 selected from dimethiconol, fluorosilicone and dimethicone and mixtures thereof; and
(ii) a silicone-based carrier having a viscosity from 0.65 mm².s⁻¹ to 100 mm².s⁻¹,
wherein the ratio of i) to ii) is from 10:90 to 20:80 and wherein said silicone component has a final viscosity of from 500 mm².s⁻¹ to about 10,000 mm².s⁻¹.

Dimethiconol-based silicones suitable for use herein have the chemical structure (II):

HO(CH₃)₂SiO[(CH₃)₂SiO]ₙ(CH₃)₂SiOH

where n is from 2000 to 40,000, preferably from 3000 to 30,000.

The fluorosilicones useful herein have a molecular weight of from 200,000 to 300,000, preferably from 240,000 to 260,000 and most preferably 250,000.

The silicone gums include dimethicones as described by Petrarch and others including US-A-4,152,416, May 1, 1979 to Spitzer, et al, and Noll, Walter, Chemistry and Technology of Silicones, New York: Academic Press 1968. Also describing silicone gums are General Electric Silicone Rubber Product Data Sheets SE 30, SE 33, SE 54 and SE 76. "Silicone gum" materials useful herein denote high molecular weight materials having a mass-average molecular weight in excess of 200,000 and preferably from 200,000 to 4,000,000. Typically, they have a viscosity at 25°C in excess of 1,000,000 mm².s⁻¹. Specific examples include polydimethylsiloxane, (polydimethylsiloxane) (methylvinylsiloxane) copolymer, poly(dimethylsiloxane) (diphenyl) (methylvinylsiloxane) copolymer and mixtures thereof.

The silicone-based carriers suitable for use herein include certain silicone fluids. The silicone fluid can be either a polyalkyl siloxane, a polyaryl siloxane, a polyalkylaryl siloxane or a polyether siloxane copolymer. Mixtures of these fluids can also be used and are preferred in certain executions.

The polyalkyl siloxane fluids that can be used include, for example, polydimethylsiloxanes with viscosities ranging from 0.65 to 600,000 mm².s⁻¹, preferably from 0.65 to 10,000 mm².s⁻¹ at 25°C. These siloxanes are available, for example, from the General Electric Company as the Viscasil (RTM) series and from Dow Corning as the Dow Corning 200 series. The essentially non-volatile polyalkylarylsiloxane fluids that can be used include, for example, polymethylphenylsiloxanes, having viscosities of about 0.65 to 30,000 mm².s⁻¹ at 25°C. These siloxanes are available, for example, from the General Electric Company as SF 1075 methyl phenyl fluid or from Dow Corning as 556 Cosmetic Grade Fluid.

Also suitable for use herein are certain volatile cyclic polydimethylsiloxanes having a ring structure incorporating from 3 to 7 (CH₃)₂SiO moieties.

The viscosity can be measured by means of a glass capillary viscometer as set forth in Dow Corning Corporate Test Method CTMOOO4, July 29, 1970. Preferably the viscosity of the silicone blend constituting the secondary oil phase ranges from 500 mm².s⁻¹ to 100,000 mm².s⁻¹, preferably from 1000 mm².s⁻¹ to 10,000 mm².s⁻¹.

The most preferred silicone component for use herein is a dimethiconol gum having a molecular weight of from 200,000 to 4,000,000 along with a silicone carrier with a viscosity of 0.65 to 100 mm².s⁻¹. An example of this silicone component is Dow Corning Q2-1403 (85% 5 mm².s⁻¹ Dimethicone Fluid/15% Dimethiconol) and Dow Corning Q2-1401 available from Dow Corning.

The silicone component is valuable herein in conjunction with the liquid-crystal forming emulsifier for modifying the perceived skin feel of the composition. Highly preferred in this respect are silicone gums having a molecular weight of from 200,000 to 4,000,000. Thus according to another aspect of the invention, there is provided a skin care composition in the form of an oil-in-water dispersion which comprises a silicone or mixture of silicones in a level of from 0.1 % to 20% by weight, the silicone or silicone mixture comprising a silicone gum having a molecular weight of from 200,000 to 4,000,000 and wherein the composition additionally incorporates from 2 % to 10 % of an emulsifier capable of forming liquid crystals in water, the emulsifier being a fatty acid ester blend based on a mixture of sorbitan fatty acid ester and sucrose fatty acid ester. The fatty acid ester in each instance is preferably C₈-C₂₄, more preferably C₁₀-C₂₀. The silicone or silicone mixture is preferably present in a level of from 0.5% to 15%, preferably from 1% to 10% by weight of composition, this level being based on the total blend of gum and non-gum silicone materials. In preferred embodiments, the compositions of this aspect of the invention comprise a primary oil phase which is essentially silicone-free together with a secondary oil phase comprising the silicone gum. The primary oil phase is described in detail above.

Preferred embodiments herein comprise from 0.1% to 10% by weight of an unsaturated fatty acid or ester. Preferred unsaturated fatty acids and esters for use herein are optionally hydroxy substituted C₈-C₅₀ unsaturated fatty acids and esters, especially esters of ricinoleic acid. The unsaturated fatty acid or ester component is valuable herein in combination with the liquid crystal forming emulsifier for improving the skin feel and rub-in characteristics of the compositon. Highly preferred in this respect is cetyl ricinoleate.

Thus according to another aspect of the invention, there is provided a skin-care composition in the form of an oil-in-water dispersion wherein the composition incorporates from 2% to 10% by weight of an emulsifier capable of forming liquid crystals in water, the emulsifier being a fatty acid ester blend based on a mixture of sorbitan fatty acid ester and sucrose fatty acid ester, and wherein the composition additionally incorporates from 0.1% to 10% of an optionally hydroxy substituted C₈-C₅₀ unsaturated fatty acid or an ester thereof, preferably an ester of ricinoleic acid.

A second essential ingredient in the composition herein is an emulsifier capable of forming liquid crystals in water. The emulsifier is preferably incorporated into the composition in an amount of from 2% to 10%, preferably from 3% to 7% by weight of composition. The emulsifier preferred for use herein is a fatty acid ester blend based on a mixture of sorbitan or sorbitol fatty acid ester and sucrose fatty acid ester, the fatty acid in each instance being preferably C₈-C₂₄, more preferably C₁₀-C₂₀. The preferred fatty acid ester emulsifier from the viewpoint of moisturisation is a blend of sorbitan or sorbitol C₁₆-C₂₀ fatty acid ester with sucrose C₁₀-C₁₆ fatty acid ester, especially sorbitan stearate and sucrose cocoate. This is commercially available from ICI under the trade name Arlatone 2121. The stabilisation mechanism of formulation derived from Arlatone 2121 is based on the formation of distinct liquid crystalline structures in the water phase into which the oil phase is dispersed. In order to achieve optimum moisturisation, absorption and skin feel together with reduced greasiness it is desirable for the ratio of primary oil to fatty acid ester emulsifier to lie in the range from 6:1 to 1:1, preferably from 4:1 to 1:1.

A wide variety of optional ingredients such as non-occlusive moisturizers, humectants, gelling agents, neutralizing agents, perfumes, colouring agents and surfactants, can be added to the skin compositions herein.

The compositions herein can comprise a humectant. Suitable humectants for use herein include sorbitol, propylene glycol, butylene glycol, hexylene glycol, ethoxylated glucose derivatives, hexanetriol, glycerine, water-soluble polyglycerylmethacrylate lubricants and panthenols. A preferred humectant herein is glycerine (sometimes known as glycerol or glycerin). Chemically, glycerine is 1,2,3-propanetriol and is a product of commerce. One large source of the material is in the manufacture of soap. Also preferred for use herein is butylene glycol.

In the present compositions, the humectant is preferably present at a level of from 0.1% to 20%, more preferably from about 1% to 10%, and especially from 2% to 5% by weight of composition.

Suitable polyglycerylmethacrylate lubricants for use in the compositions of this invention are available under the trademark Lubrajel (RTM) from Guardian Chemical Corporation, 230 Marcus Blvd., Hauppage, N.Y. 11787. In general, Lubrajels can be described as hydrates or clathrates which are formed by the reaction of sodium glycerate with a methacrylic acid polymer. Thereafter, the hydrate or clathrate is stabilized with a small amount of propylene glycol, followed by controlled hydration of the resulting product. Lubrajels are marketed in a number of grades of varying glycerate: polymer ratio and viscosity. Suitable Lubrajels include Lubrajel TW, Lubrajel CG and Lubrajel MS, Lubrajel WA, Lubrajel DV and so-called Lubrajel Oil.

The compositions of the invention can also contain a hydrophilic gelling agent at a level preferably from 0.01% to 10%, more preferably from 0.02% to 2%, and especially from 0.02% to 0.5%. The gelling agent preferably has a viscosity (1 % aqueous solution, 20°C, Brookfield RVT) of at least 4000 mPa.s, more preferably at least 10,000 mPa.s and especially at least 50,000 mPa.s.

Suitable hydrophilic gelling agents can generally be described as water-soluble or colloidally water-soluble polymers, and include cellulose ethers (e.g. hydroxyethyl cellulose, methyl cellulose, hydroxypropylmethyl cellulose), polyvinylpyrrolidone, polyvinylalcohol, guar gum, hydroxypropyl guar gum and xanthan gum.

Preferred hydrophilic gelling agents herein, however, are acrylic acid/ethyl acrylate copolymers and the carboxyvinyl polymers sold by the B.F. Goodrich Company under the trade mark of Carbopol resins. These resins consist essentially of a colloidally water-soluble polyalkenyl polyether crosslinked polymer of acrylic acid crosslinked with from 0.75% to 2.00% of a crosslinking agent such as for example polyallyl sucrose or polyallyl pentaerythritol. Examples include Carbopol 934, Carbopol 940, Carbopol 950, Carbopol 980, Carbopol 951 and Carbopol 981. Carbopol 934 is a water-soluble polymer of acrylic acid crosslinked with 1% of a polyallyl ether of sucrose having an average of about 5.8 allyl groups for each sucrose molecule. A most preferred polymer is Carbopol 951. Also suitable for use herein are hydrophobically-modified cross-linked polymers of acrylic acid having amphipathic properties available under the Trade Name Carbopol 1382, Carbopol 1342 and Pemulen TR-1 (CTFA Designation: Acrylates/10-30 Alkyl Acrylate Crosspolymer). A combination of the polyalkenyl polyether cross-linked acrylic acid polymer and the hydrophobically modified cross-linked acrylic acid polymer is also suitable and is preferred for use herein. The gelling agents herein are particularly valuable for providing excellent stability characteristics over both normal and elevated temperatures. Neutralizing agents suitable for use in neutralizing acidic group containing hydrophilic gelling agents herein include sodium hydroxide, potassium hydroxide, ammonium hydroxide, monoethanolamine, diethanolamine and triethanolamine.

The compositions of the invention are in emulsion form and are preferably formulated so as to have a product viscosity of at least about 4,000 mPa.s and preferably in the range from 4,000 to 300,000 mPa.s, more preferably from 8,000 to 200,000 mPa.s and especially from 10,000 to 50,000 mPa.s (25°C, neat, Brookfield RVT Spindle No. 5).

The compositions of the invention can also contain from 0.1% to 10%, preferably from 1% to 5% of a panthenol moisturizer. The panthenol moisturizer can be selected from D-panthenol ([R]-2,4-dihydroxy-N-[3-hydroxypropyl)]-3,3-dimethylbutamide), DL-panthenol, calcium pantothenate, royal jelly, panthetine, pantotheine, panthenyl ethyl ether, pangamic acid, pyridoxin, pantoyl lactose and Vitamin B complex. Highly preferred from the viewpoint of skin care and tack reduction is D-panthenol.

The compositions of the present invention can additionally comprise from 0.001 % to 0.5%, preferably from 0.002% to 0.05%, more preferably from 0.005% to 0.02% by weight of carboxymethylchitin. Chitin is a polysaccharide which is present in the integument of lobsters and crabs and is a mucopolysaccharide having beta (1-4) linkages of N-acetyl-D-glucosamine. Carboxymethylchitin is prepared by treating the purified chitin material with alkali followed by monochloracetic acid. It is sold commercially in the form of a dilute (approximately 0.1% to 0.5% by weight) aqueous solution under the name Chitin Liquid available from A & E Connock Ltd., Fordingbridge, Hampshire, England.

Other optional materials include keratolytic agents such as salicylic acid; proteins and polypeptides and derivatives thereof; water-soluble or solubilizable preservatives such as Germall 115, methyl, ethyl, propyl and butyl esters of hydroxybenzoic acid, benzyl alcohol, EDTA, Euxyl (RTM) K400, Bromopol (2-bromo-2-nitropropane-1,3-diol) and phenoxypropanol; anti-bacterials such as Irgasan (RTM) and phenoxyethanol (preferably at levels of from 0.1% to 5%); soluble or colloidally-soluble moisturising agents such as hylaronic acid and starch-grafted sodium polyacrylates such as Sanwet (RTM) IM-1000, IM-1500 and IM-2500 available from Celanese Superabsorbent Materials, Portsmith, VA, USA and described in USA-A-4,076,663; colouring agents; perfumes and perfume solubilizers and additional surfactants/emulsifiers such as fatty alcohol ethoxylates, ethoxylated polyol fatty acid esters, wherein the polyol can be selected from glycerine, propyleneglycol, ethyleneglycol, sorbitol, sorbitan, polypropyleneglycol, glucose and sucrose. Examples include glyceryl monohydroxy stearate and stearyl alcohol ethoxylated with an average of from 10 to 200 moles of ethyleneoxide per mole of alcohol and PEG-6 caprylic/capric glycerides.

Preferred embodiments of the invention additionally comprise from 0.1% to 5% by weight of aluminium starch octenylsuccinate. Aluminium starch octenylsuccinate is the aluminium salt of the reaction product of octenylsuccinic anhydride with starch and is commercially available under the trade name from Dry Flo National Starch & Chemical Ltd. Dry Flo is useful herein from the viewpoint of skin feel and application characteristics.

Other optional materials herein include pigments. Pigments suitable for use in the compositions of the present invention can be organic and/or inorganic. Also included within the term pigment are materials having a low colour or lustre such as matte finishing agents, and also light scattering agents. Examples of suitable pigments are iron oxides, acyglutamate iron oxides, ultramarine blue, D&C dyes, carmine, and mixtures thereof. Depending upon the type of composition, a mixture of pigments will normally be used. The preferred pigments for use herein from the viewpoint of moisturisation, skin feel, skin appearance and emulsion compatibility are treated pigments. The pigments can be treated with compounds wuch as amino acids, silicones, lecithin and ester oils.

The pH of the compositions is preferably from 4 to 9, more preferably from 5 to 7.5.

The invention is illustrated by the following examples

| Examples I to V | | | | | |
|---|---|---|---|---|---|
| | I | II | III | IV | V |
| Cetyl Alcohol | 0.25 | 0.3 | 0.2 | 0.3 | 0.25 |
| Stearic Acid | 0.11 | 0.2 | 0.1 | 0.2 | 0.1 |
| Steareth 100 | 0.1 | 0.1 | 0.15 | 0.15 | 0.15 |
| GMHS (1) | 0.15 | 0.2 | 0.1 | 0.2 | 0.15 |
| Cetyl Palmitate | 3.0 | 2 | 3 | 4 | 2.5 |
| Mineral Oil | 2.0 | 3 | 4 | 3 | 3.5 |
| Petrolatum | 3.00 | 2 | 2.5 | 4 | 3.5 |
| Wickenol 142 (RTM) | 0.60 | 1 | 1 | 1 | 0.7 |
| Dimethicone 200 | 0.3 | 0.4 | 0.5 | 0.5 | 0.4 |
| Propyl Paraben | 0.08 | 0.08 | 0.07 | 0.08 | 0.07 |
| Arlatone (RTM) 2121 | 6 | 4 | 7 | 5 | 4 |
| Glycerin | 3 | 3 | 3 | 2 | 3 |
| Carbopol (RTM) 1342 | 0.095 | 0.075 | 0.075 | 0.075 | 0.075 |
| Carbopol (RTM) 951 | 0.09 | 0.08 | 0.09 | 0.09 | 0.08 |
| Na4 EDTA | 0.1 | 0.2 | 0.1 | 0.1 | 0.1 |
| Methyl Paraben | 0.175 | 0.175 | 0.175 | 0.175 | 0.175 |
| KOH | 0.3 | 0.2 | 0.2 | 0.2 | 0.2 |
| Dimethicone Q21403 | 3 | - | 4 | 3 | - |
| CeRiccinoleate | - | 2 | - | 1 | - |
| Butylene Glycol | - | - | - | 2 | - |
| DryFlo (RTM) | - | 1 | - | 0.5 | - |
| Perfume | 0.2 | 0.2 | - | 0.2 | - |
| Colour | 0.0004 | 0.0002 | 0.0003 | - | - |

### 1. Glycerylmonohydroxystearate

The compositions are made as follows:

A first premix of thickening agents, Arlatone 2121 and other water soluble ingredients is prepared by admixing in water and heating. A second premix of oil phase ingredients other than silicone gum is prepared by mixing and heating and is added to the aqueous premix.

The resulting mixture is cooled. The silicone gum is then added to the resulting oil-in-water emulsion and the mixture is cooled before adding minor ingredients. The composition is ready for packaging.

The compositions display improved moisturisation, skin feel and skin care characteristics together with reduced greasiness and excellent rub-in and absorption characteristics.

## Claims

1. A skin care composition in the form of an oil-in-water dispersion comprising one or more distinct oil phases wherein the primary oil phase is present in a level of from 4% to 11% by weight and wherein the composition incorporates from 2% to 10% by weight of an emulsifier capable of forming liquid crystals in water, the emulsifier being a fatty acid ester blend based on a mixture of sorbitan fatty acid ester and sucrose fatty acid ester, and wherein the ratio of primary oil phase to fatty acid ester emulsifier is in the range from 6:1 to 1:1.

2. A skin care composition according to Claim 1 where the emulsifier is a blend of sorbitan stearate and sucrose cocoate.

3. A skin care composition according to Claim 1 or 2 wherein the ratio of primary oil phase to fatty acid ester emulsifier is in the range from 4:1 to 1:1.

4. A skin care composition according to any of Claims 1 to 3 comprising from 5% to 11% by weight of primary oil phase.

5. A skin care composition according to any of Claims 1 to 4 comprising from 3% to 7% by weight of emulsifier.

6. A skin care composition according to any of Claims 1 to 5 wherein the primary oil phase comprises a natural or synthetic oil selected from mineral, vegetable and animal oils, fats and waxes, fatty acid esters, fatty alcohols, fatty acids and mixtures thereof.

7. A skin care composition according to any of Claims 1 to 6 additionally comprising a secondary oil phase which comprises from 0.1% to 20% by weight of composition of a silicone or silicone mixture which includes a silicone gum having a molecular weight of from 200,000 to 4,000,000.

8. A skin care composition according to any of Claims 1 to 7 additionally comprising from 0.1% to 10% of an optionally hydroxy substituted C₈-C₅₀ unsaturated fatty acid or an ester thereof, preferably an ester of riccinoleic acid.

9. A skin care composition according to any of Claims 1 to 8 additionally comprising from 0.1 % to 20% by weight of a humectant selected from glycerine, polyglycerylmethacrylate lubricants, butylene glycol, sorbitol, panthenols, propylene glycol, hexylene glycol, ethoxylated glucose derivatives, hexanetriol and glucose ethers, and mixtures thereof.

10. A skin care composition according to any of Claims 1 to 9 wherein the humectant is selected from glycerine and butylene glycol, and mixtures thereof

11. A skin care composition according to any of Claims 1 to 10 additionally comprising from 0.01% to 10% by weight of a hydrophilic gelling agent selected from carboxyvinyl polymers, preferably colloidally water-soluble polymers of acrylic acid cross-linked with from 0.75% to 2% of a cross-linked agent selected from polyalkyl sucrose and polyalkyl pentaerythritol.

12. A skin-care composition according to any of Claims 1 to 11 additionally comprising from 0.1% to 5% by weight of aluminium starch octenylsuccinate.

## Patentansprüche

1. Hautpflegezusammensetzung in Form einer Öl-in-Wasser-Dispersion, umfassend eine oder mehrere getrennte Ölphasen, wobei die primäre Ölphase in einem Anteil von 4 bis 11 Gew.-% vorliegt, und wobei die Zusammensetzung 2 bis 10 Gew.-% eines Emulgiermittels beinhaltet, das in der Lage ist, Flüssigkristalle in Wasser zu bilden, wobei das Emulgiermittel eine Fettsäureestermischung ist, basierend auf einer Mischung aus Sorbitanfettsäureester und Saccharosefettsäureester, und wobei das Verhältnis von primärer Ölphase zu Fettsäureester-Emulgiermittel im Bereich von 6:1 bis 1:1 liegt.

2. Hautpflegezusammensetzung nach Anspruch 1, wobei das Emulgiermittel eine Mischung aus Sorbitanstearat und und Saccharosecocoat ist.

3. Hautpflegezusammensetzung nach Anspruch 1 oder 2, wobei das Verhältnis von primärer Ölphase zu Fettsäureester-Emulgiermittel im Bereich von 4:1 bis 1:1 liegt.

4. Hautpflegezusammensetzung nach mindestens einem der Ansprüche 1 bis 3, umfassend 5 bis 11 Gew.-% primärer Ölphase.

5. Hautpflegezusammensetzung nach mindestens einem der Ansprüche 1 bis 4, umfassend 3 bis 7 Gew.-% Emulgiermittel.

6. Hautpflegezusammensetzung nach mindestens einem der Ansprüche 1 bis 5, wobei die primäre Ölphase ein natürliches oder synthetisches Öl umfaßt, gewählt aus mineralischen, pflanzlichen und tierischen Ölen, Fetten und Wachsen, Fettsäureestern, Fettalkoholen, Fettsäuren und Mischungen hiervon.

7. Hautpflegezusammensetzung nach mindestens einem der Ansprüche 1 bis 6, umfassend zusätzlich eine sekundäre Ölphase, welche 0,1 bis 20 Gew.-% einer Zusammensetzung aus einem Silicon oder einer Siliconmischung umfaßt, welche einen Silicongummi mit einem Molekulargewicht von 200.000 bis 4.000.000 beinhaltet.

8. Hautpflegezusammensetzung nach mindestens einem der Ansprüche 1 bis 7, umfassend zusätzlich 0,1 bis 10% einer wahlweise Hydroxysubstituierten C₈-C₅₀ ungesättigten Fettsäure oder einen Ester hiervon, vorzugsweise einen Ester von Ricinolsäure.

9. Hautpflegezusammensetzung nach mindestens einem der Ansprüche 1 bis 8, umfassend zusätzlich 0,1 bis 20 Gew.-% eines Feuchthaltemittels, gewählt aus Glycerin, Polyglycerylmethacrylat-Gleitmitteln, Butylenglykol, Sorbitol, Panthenolen, Propylenglykol, Hexylenglykol, ethoxylierten Glucosederivaten, Hexantriol und Glucoseethern und Mischungen hiervon.

10. Hautpflegezusammensetzung nach mindestens einem der Ansprüche 1 bis 9, wobei das Feuchthaltemittel aus Glycerin und Butylenglykol und Mischungen hiervon gewählt ist.

11. Hautpflegezusammensetzung nach mindestens einem der Ansprüche 1 bis 10, umfassend zusätzlich 0,01 bis 10 Gew.-% eines hydrophilen Geliermittels, gewählt aus Carboxyvinylpolymeren, vorzugsweise kolloidalen, wasserlöslichen Polymeren von Acrylsäure, vernetzt mit 0,75 bis 2% eines Vernetzungsmittels, gewählt aus Polyalkylsaccharose und Polyalkylpentaerythritol.

12. Hautpflegezusammensetzung nach mindestens einem der Ansprüche 1 bis 11, umfassend zusätzlich 0,1 bis 5 Gew.-% Aluminiumstärkeoctenylsuccinat.

## Revendications

1. Composition de soin de la peau sous la forme d'une dispersion huile-dans-eau, comprenant une ou plusieurs phases huileuses distinctes, dans laquelle la phase huileuse principale est présente dans une quantité comprise entre 4 % et 11 % en poids, et dans laquelle la composition comprend 2 % à 10 % en poids d'un émulsifiant capable de former des cristaux liquides dans l'eau, l'émulsifiant étant un mélange d'esters d'acides gras reposant sur un mélange d'ester d'acide gras de sorbitan et d'ester d'acide gras de saccharose, et dans laquelle le rapport entre la phase huileuse principale et l'émulsifiant d'ester d'acide gras est compris dans la gamme de 6:1 à 1:1.

2. Composition de soin de la peau selon la revendication 1, dans laquelle l'émulsifiant est un mélange de stéarate de sorbitan et de cocoate de saccharose.

3. Composition de soin de la peau selon la revendication 1 ou 2, dans laquelle le rapport entre la phase huileuse principale et l'émulsifiant d'ester d'acide gras est compris dans la gamme de 4:1 à 1:1.

4. Composition de soin de la peau selon l'une quelconque des revendications 1 à 3, comprenant 5 % à 11 % en poids d'une phase huileuse principale.

5. Composition de soin de la peau selon l'une quelconque des revendications 1 à 4, comprenant 3 % à 7 % en poids d'un émulsifiant.

6. Composition de soin de la peau selon l'une quelconque des revendications 1 à 5, dans laquelle la phase huileuse principale comprend une huile naturelle ou synthétique choisie parmi les huiles minérales, végétales et animales, les graisses et les cires, les esters d'acides gras, les alcools gras, les acides gras et des mélanges de ceux-ci.

7. Composition de soin de la peau selon l'une quelconque des revendications 1 à 6, comprenant, en outre, une phase huileuse secondaire qui contient 0,1 % à 20 % en poids de la composition d'une silicone ou d'un mélange de silicones comprenant de la gomme de silicone présentant un poids moléculaire compris entre 200 000 et 4 000 000.

8. Composition de soin de la peau selon l'une quelconque des revendications 1 à 7, comprenant, en outre, 0,1 % à 10 % d'un acide gras insaturé en C₈ à C₅₀ éventuellement substitué par un hydroxy, ou d'un ester de celui-ci, de préférence un ester de l'acide ricinoléique.

9. Composition de soin de la peau selon l'une quelconque des revendications 1 à 8, comprenant, en outre, 0,1 % à 20 % en poids d'un humectant choisi parmi la glycérine, les lubrifiants de méthacrylate de polyglycéryle, le butylèneglycol, le sorbitol, les panthénols, le propylèneglycol, l'hexylèneglycol, les dérivés de glucose éthoxylés, l'hexanetriol et les éthers de glucose, et des mélanges de ceux-ci.

10. Composition de soin de la peau selon l'une quelconque des revendications 1 à 9, dans laquelle l'humectant est choisi parmi la glycérine et le butylèneglycol, et des mélanges de ceux-ci.

11. Composition de soin de la peau selon l'une quelconque des revendications 1 à 10, comprenant, en outre, 0,01 % à 10 % en poids d'un agent gélifiant hydrophile choisi parmi les polymères de carboxyvinyle, de préférence les polymères hydrosolubles par colloïdalité d'acide acrylique réticulés avec 0,75 % à 2 % d'un agent réticulé choisi parmi le polyalkylsaccharose et le polyalkylpentaérythritol.

12. Composition de soin de la peau selon l'une quelconque des revendications 1 à 11, comprenant, en outre, 0,1% à 5% en poids d'octénylsuccinate d'amidon et d'aluminium.
